# EUROPEAN PATENT SPECIFICATION

(11) **EP 1 622 882 B1**
(45) Date of publication and mention of the grant of the patent: **12.07.2006**
(21) Application number: 04732927.1
(22) Date of filing: 14.05.2004
(51) Int. Cl.: C07D 257/04

(54) **A METHOD OF PREPARATION OF N -(1-OXOPENTYL)- N-¬¬ 2-(1TETRAZOL-5-y1) ¬1,1 -BIPHENYL|-4-y1 METHYL| -L-VALINE (VALSARTAN)**
VERFAHREN ZUR HERSTELLUNG VON N-(1-OXOPENTYL)-N-¬¬2'-(1H-TETRAZOL-5-YL)¬1,1'-BIPHENYL|-4-YL|METHYL|-L-VALINE (VALSARTAN)
PROCEDE DE PREPARATION DE N -(1-OXOPENTYL)- N- ¬¬2'-(1H -TETRAZOL-5-Y1) 1,1'-BIPHENYL|-4-Y1|METHYL|-L-VALINE (VALSARTAN)

(30) Priority: 15.05.2003 CZ 20031360
(43) Date of publication of application: 08.02.2006
(73) Proprietor: Zentiva, a.s., 102 37 Praha 10 (CZ)
(72) Inventor: RADL, Stanislav, 143 00 Praha 4 (CZ); STACH, Jan, 190 16 Praha 9 - Ujezd nad Lesy (CZ); DEDINOVA, Eva, 109 00 Praha 10-Horni Mecholupy (CZ)
(74) Representative: Jirotkova, Ivana
(86) International application number: PCT/CZ2004/000029
(87) International publication number: WO 2004/101534

(56) References cited:
- US-A- 5 399 578

## Description

### Technical Field

This invention concerns an improved method of preparation of *N*-(1-oxopentyl)-*N*-[[2'-(1*H-*tetrazol-5-yl)[1,1'-biphenyl]-4-yl]methyl]-L-valine, known under the INN name valsartan, formula I

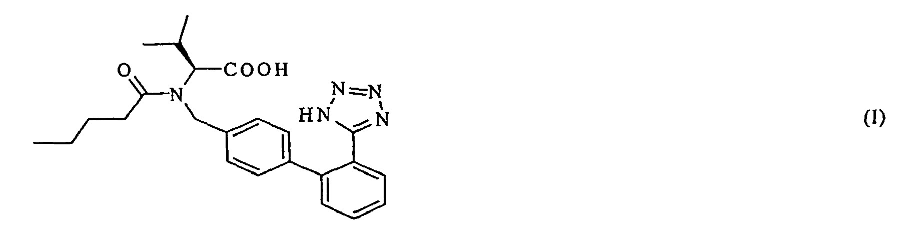

The mentioned drug belongs to the group of drugs known as antagonists of angiotensin II receptor, which helps to regulate high blood pressure.

### Background Art

Valsartan of formula I is produced according to the published patent (US patent No. 5,399,578) via the following methods:

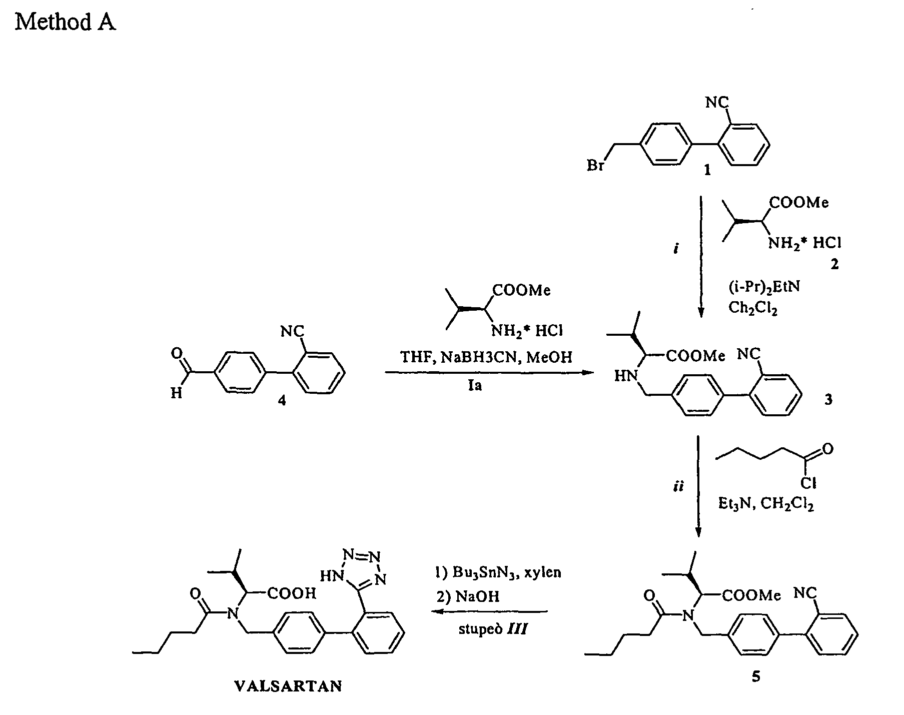

Synthesis starts with the bromo derivative 1 and the methyl ester of L-valine (2), whereas the key step is building of the tetrazol ring with tributyl stannyl azide. The cited US patent further describes a variant of this method with use of the benzyl ester of L-valine wherein the benzyl group is removed via catalytic hydrogenation.

A disadvantage of the above-mentioned methods is the use of toxic tributyl stannyl azide to build the tetrazol ring and high demands on security in order to prevent an explosion because hydrogen azide develops during the step.

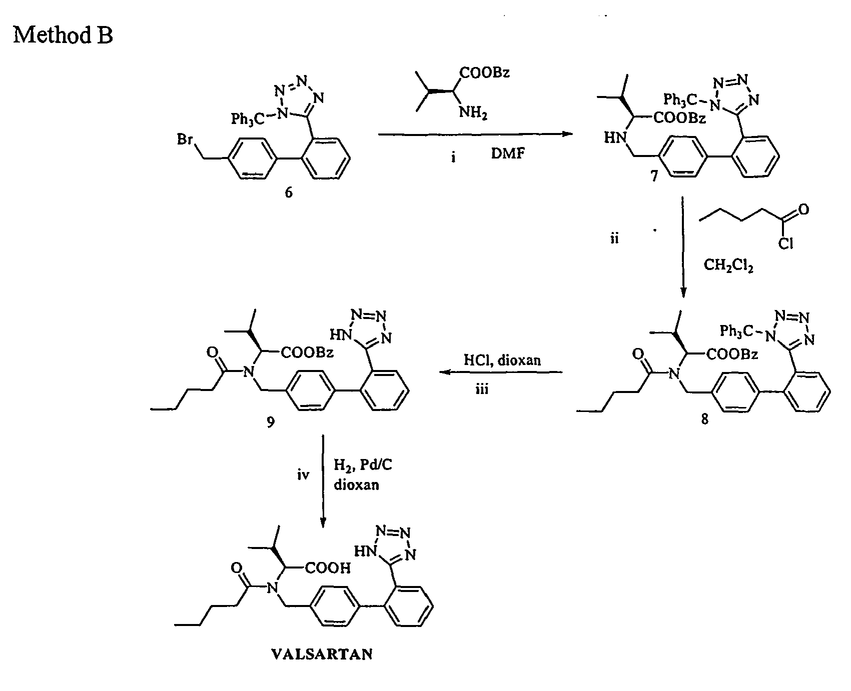

Method B starts with commercially available 4-bromomethyl-2'-(1-triphenylmethyltetrazol-5-yl)biphenyl (6). A disadvantage of the method is the fact that all the intermediates except the compound 9 are oily substances that cannot be crystallized. The final product is, therefore, strongly contaminated and the required repeated crystallization results in a significant loss of yield.

An accurate reproduction of processes according to USP 5,399,578 is rather problematic. Method B, wherein the above-mentioned free base is used, documented in Example 55, is quite without data on weighed quantities in two steps, and in the case of said fee base (Example 55a) it is even referred to analogous Example 57a, which does not relate to the given reaction at all. Moreover, synthesis in this example is not finished up to valsartan, but only to its benzyl ester. When using a procedure analogous to that of Example 55, we were obtaining only 85% purity of valsartan, which had to be re-crystallized many times up to the desired purity, which entails increased amounts of raw materials used and also of labour, which involves elevated costs. Industrially, the method is practically not feasible.

### Disclosure of Invention

The subject matter of this invention is an improved method of preparation of *N*-(1-oxopentyl)-*N*-[[2'-(1*H*,tetrazol-5-yl)[1,1'-biphenyl]-4-yl]methyl]-L-valine of formula I, known under the INN name valsartan. The essence consists in isolation of intermediate product 7, used in the above mentioned method B, in the form of its hydrochloride in a very pure form.

A detailed description of the invention follows:

The starting compound of the method B, the bromo derivative 6, provides, in subsequent reaction with L-valine benzyl ester, a product of purity of only about 80%. Use of such intermediate, in turn, results in contaminated valsartan, which has to be re-crystallized many times, which entails elevated economic costs. It is not easy to synthesize the hydrochloride of compound 7 because the trityl protecting group is instable in acid environment and any excess of hydrogen chloride - especially at higher temperature - leads gradually to detritylation of the product. In our method, the hydrochloride of formula III 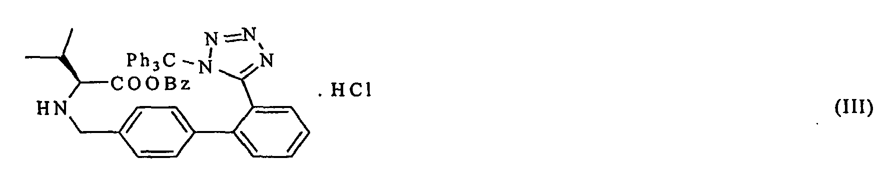 is obtained via acidification of the reaction mixture with hydrochloric acid after the reaction of compound 6 with L-valine benzyl ester in acetonitrile and extraction of the hydrochloride with a suitable solvent, preferably with ethyl acetate. After washing the extract with water, in which hydrochloride III is completely insoluble, the crystalline product having purity above 90% is obtained via gradual dehydration of the extract after adding toluene or via addition of a non-polar solvent, preferably hexane. Hydrochloride III can also be obtained via transferring the free base into ethyl acetate or toluene and adding an equivalent of hydrochloric acid, gaseous hydrogen chloride or adding hydrogen chloride dissolved in a suitable solvent, preferably methanol.

The invention is explained in more detail in the following working examples. The examples, which illustrate improvement in the method according to the invention, have a purely illustrative character and do not limit the extent of the invention in any respect.

### Examples

### Example 1

### N-[(2'-(1-Triphenylmethyl-tetrazol-5-yl)biphenyl-4-yl)methyl]-(L)-valine benzyl ester hydrochloride (III)

4-Bromomethyl-2'-(1-triphenylmethyltetrazol-5-yl)biphenyl (5 g, 9 mmol), L-valine benzyl ester 4-toluenesulfonate (3.4 g, 9 mmol) and ethyl diisopropyl amine (3.8 ml) were refluxed in acetonitrile for 2 hours. After cooling down, the mixture was diluted with ethyl acetate (40 ml), demineralized water (20 ml) and acidified with hydrochloric acid to pH 1. After separating the resulting layers, the organic phase was washed with water (4 x 5 ml) and after adding 3 ml of toluene, it was evaporated, mixed in ethyl acetate (10 ml) at 40 °C. After 2 ml of hexane was added, the product crystallized out; 4.62 g (72 %). Melting point 107 °C-111 °C. The product purity was above 90%.

### Example 2

### N-[(2'-(1-Triphenylmethyl-tetrazol-5-yl)biphenyl-4-yl)methyl]-(L)-valine benzyl ester hydrochloride (III)

4-Bromomethyl-2'-(1-triphenylmethyltetrazol-5-yl)biphenyl (5 g, 9 mmol), L-valine benzyl ester 4-toluenesulfonate (3.4 g, 9 mmol) and ethyl diisopropyl amine (3.8 ml) were refluxed in acetonitrile for 2 hours. After cooling down, acetonitrile was evaporated in a vacuum evaporator, the resulting mixture was diluted with ethyl acetate (20 ml), washed with water (2 x 5 ml) and concentrated to a volume of 10 ml. After adding an equivalent of concentrated hydrochloric acid and cooling down to -15 °C, the product crystallized out; 4.0 g (62 %). Melting point 107°C-111°C. The product purity was above 90%.

### Example 3

### N-[(2'-(1-Triphenylmethyl-tetrazol-5-yl)biphenyl-4-yl)methyl]-(L)-valine benzyl ester hydrochloride (III)

4-Bromomethyl-2'-(1-triphenylmethyltetrazol-5-yl)biphenyl (5.29 kg), L-valine benzyl ester 4-toluenesulfonate (3.6 kg) and ethyl diisopropyl amine (41) were refluxed in acetonitrile (241) for 2 h. After evaporating acetonitrile (HPLC of the fee base: 79.7%) the mixture was diluted with ethyl acetate (24 1), washed with demineralized water (2 x 41) and, after cooling down to 5 °C, concentrated hydrochloric acid (0.83 kg) was added dropwise. After sucking-off and drying the product, 5.7 kg (84 %) of the product were obtained. Melting point 107 °C-111 °C, HPLC 92%.

### Example 4

### N-[(2'-(1-Triphenylmethyl-tetrazol-5-yl)biphenyl-4-yl)methyl]-N-valeryl-(L)-valine benzyl ester

*N*-[(2'-(1-Triphenylmethyl-tetrazol-5-yl)biphenyl-4-yl)methyl]-(L)-valine benzyl ester hydrochloride (III) (625 g) was mixed in toluene (2800 ml) and *N*,*N*-diisopropyl ethyl amine (490 ml) was added at 25 °C. After stirring for 30 minutes, it was cooled down to 4 °C and valeryl chloride (203 g) was added under cooling to 4-10 °C during 1.5 h. After a TLC control 50 ml of water were added and stirring was continued for another 30 min with stopped cooling. The raw mixture was filtered, the precipitate on the filter was washed with toluene (300 ml) and the resulting solution was washed with water (2 x 250 ml). After drying with magnesium sulfate (50 g) it was filtered and evaporated, yielding 800 g of dark red oily substance.

### Example 5

### N-(1-Oxopentyl)-N-[[2'-(1H-tetrazol-5-yl)[1,1'-biphenyl]-4-yl]methyl]-(L)-valine (valsartan)

*N*-[(2'-(1-Triphenylmethyl-tetrazol-5-yl)biphenyl-4-yl)methyl]-*N*-valeryl-(L)-valine benzyl ester (660 g) was dissolved under boiling in methanol (1400 ml) and the mixture was refluxed still for 8 h. After carrying out the detritylation, a Pd/C catalyst (75 g, 3 %, 50% water) was added, and the mixture was hydrogenated (1 atm) at 40 °C for 16 h. After checking by TLC the mixture was filtered and evaporated in vacuo. Isopropanol (200 ml) and demineralized water (800 ml) were added, the pH was adjusted to 8 by addition of 10% KOH (1350 ml). It was extracted with toluene (2 x 750 ml) and after adding ethyl acetate (1250 ml) the aqueous phase was acidified with 36% HCl (200 ml) to pH=1. After separating the organic layer the aqueous phase was extracted with ethyl acetate (600 ml) once more. The combined organic phases were washed with demineralized water (2 x 250 ml) and evaporated at bath temperature of 50 °C with azeotropic separation of water. The raw product was dissolved in ethyl acetate (1200 ml) at 50 °C and cyclohexane (2500 ml) was added dropwise to the resulting solution during 2 h, while the substance crystallized; after cooling down to 10 °C it was sucked off and drying in vacuo at 50 °C yielded 250 g (67%) of valsartan; HPLC 98.3 %.

## Claims

1. A method of preparation of *N*-(1-oxopentyl)-*N*-[[2'-(1*H*-tetrazol-5-yl)[1,1'-biphenyl]-4-yl]methyl]-L-valine, known under the INN name valsartan, of formula I 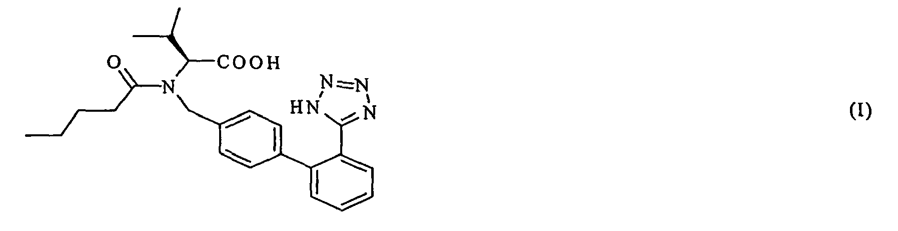 in which *N*-[(2'-(1-triphenylmethyl-tetrazol-5-yl)biphenyl-4-yl)methyl]-(L)-valine benzyl ester is obtained from the starting 4-bromomethyl-2'-(1-triphenylmethyltetrazol-5-yl)biphenyl via a reaction with L-valine benzyl ester, and is converted to valsartan via a reaction with valeryl chloride and removing the protective groups,
**characterized in that** *N*-[(2'-(1-triphenylmethyl-tetrazol-5-yl)biphenyl-4-yl)methyl]-(L)-valine benzyl ester of formula 7 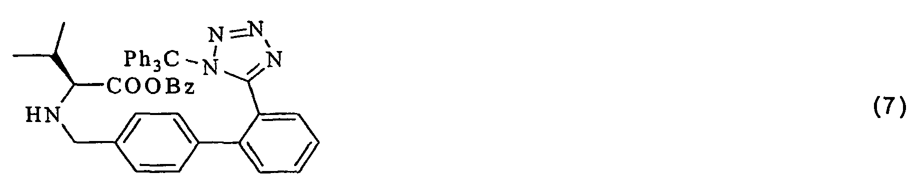 obtained in the first stage, is converted, with hydrochloric acid, to *N*-[(2'-(1-triphenylmethyltetrazol-5-yl)biphenyl-4-yl)methyl]-(L)-valine benzyl ester hydrochloride of formula III 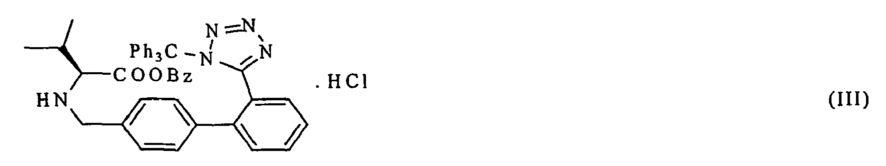 which is optionally re-crystallized.

2. The method according to claim 1 **characterized in that** the compound of formula III is prepared via acidification of a solution of *N*-[(2'-(1-triphenylmethyl-tetrazol-5-yl)biphenyl-4-yl)methyl]-(L)-valine benzyl ester base in an aprotic solvent with hydrochloric acid or hydrogen chloride with simultaneous extraction of the resulting hydrochloride of formula III.

3. The method according to claim 2 **characterized in that** the compound of formula III is extracted with ethyl acetate and subsequently crystallized therefrom.

4. The method according to claim **1 characterized in that** the free base of *N*-[(2'-(1-triphenylmethyl-tetrazol-5-yl)biphenyl-4-yl)methyl]-(L)-valine benzyl ester of formula 7 is transferred into ethyl acetate or toluene and subsequently, an equivalent of hydrochloric acid, gaseous hydrogen chloride or hydrogen chloride dissolved in an organic solvent, preferably methanol, is added, and the compound of formula III is crystallized therefrom.

5. The method according to claims 3 or 4 **characterized in that** crystallization of the compound III is performed via removing water from the solution or adding a non-polar solvent or cooling down the solution, optionally by combination of the mentioned methods.

6. The method according to claim 5, **characterized in that** a C5 up to C8 aliphatic or alicyclic hydrocarbon, or optionally a C6 up to C9 aromatic hydrocarbon, or optionally a C4 up to C8 ether, is used as the non-polar solvent.

7. The method according to claim 6, **characterized in that** pentane, hexane, cyclohexane, toluene or diethyl ether is used as the non-polar solvent.

8. The compound *N*-[(2'-(1-triphenylmethyl-tetrazol-5-yl)biphenyl-4-yl)methyl]-(L)-valine benzyl ester hydrochloride of formula III

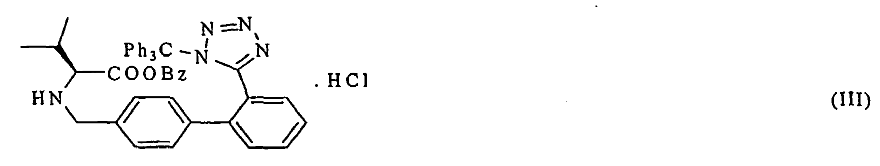

9. The compound according to claim 8, wherein the compound is in the crystalline state.

## Patentansprüche

1. Verfahren zur Herstellung von *N*-(1-Oxopentyl)-N-[[2'-(1*H*-tetrazol-5-yl)[1,1'-biphenyl]-4-yl]methyl]-L-valin, bekannt unter dem INN Namen Valsartan, der Formel I 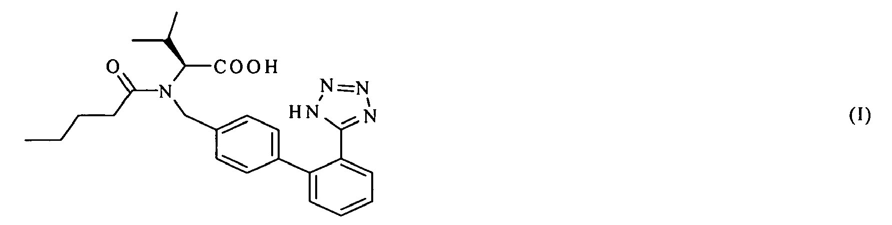 worin der *N*-[(2'-(1-Triphenyhmethyl-tetrazol-5-yl)biphenyl-4-yl)methyl]-(L)-valinbenzylester aus dem 4-Brommethyl-2'-(1-triphenylmethyltetrazol-5-yl)biphenyl durch Umsetzung mit dem L-Valinbenzylester hergestellt wird und durch Umsetzung mit Valerylchlorid und Entfernung der Schutzgruppen in Valsartan umgeformt wird, **dadurch gekennzeichnet, dass** der *N*-[(2'-(1-Triphenylmethyl-tetrazol-5-yl)biphenyl-4-yl)methyl]-(L)-valinbenzylester der Formel 7 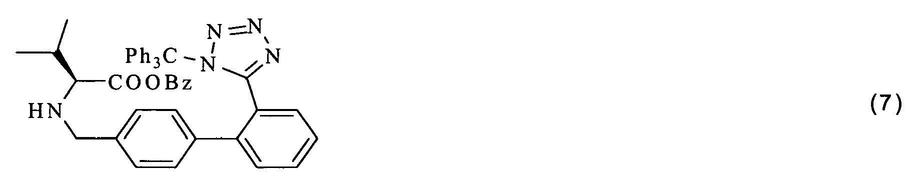 hergestellt in der ersten Stufe, mit der Chlorwasserstoffsäure in das *N*-[(2'-(1-Triphenylmethyl-tetrazol-5-yl)biphenyl-4-yl)methyl]-(L)-valinbenzylesterhydrochlorid der Formel III 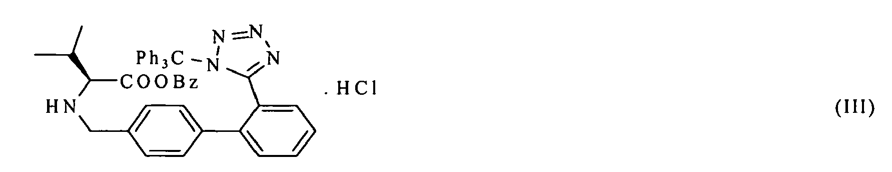 umgeformt wird, welches eventuell rekristallisiert wird.

2. Verfahren nach Anspruch 1, **dadurch gekennzeichnet, dass** die Verbindung der Formel III hergestellt wird durch die Versauerung der Lösung von der Base des *N*-[(2'-(1-Triphenylmethyl-tetrazol-5-yl)biphenyl-4-yl)methyl]-(L)-valinbenzylesters in einem aprotischen Lösungsmittel mit der Chlorwasserstoffsäure oder Chlorwasserstoff mit simultanen Extraktion des resultierenden Hydrochlorids der Formel III.

3. Verfahren nach Anspruch 2, **dadurch gekennzeichnet, dass** die Verbindung der Formel III mit Ethylacetat extrahiert und dann daraus kristallisiert wird.

4. Verfahren nach Anspruch 1, **dadurch gekennzeichnet, dass** die freie Base von *N*-[(2'-(1-Triphenylmethyl-tetrazol-5-yl)biphenyl-4-yl)methyl]-(L)-valinbenzylester der Formel 7 in Ethylacetat oder Toluol übertragen wird und dann wird ein Äquivalent der Chlorwasserstoffsäure, gasförmigen Chlorwasserstoff oder Chlorwasserstoff gelöst in einem organischen Lösungsmittel, vorzugsweise Methanol, zugegeben und die Verbindung der Formel III daraus kristallisiert.

5. Verfahren nach Anspruch 3 oder 4, **dadurch gekennzeichnet, dass** die Kristallisation der Verbindung III durch Entfernen des Wassers von der Lösung oder durch Zusatz eines nichtpolaren Lösungsmittels oder durch Kühlung, bzw. durch Kombination dieser Methoden durchgeführt wird.

6. Verfahren nach Anspruch 5, **dadurch gekennzeichnet, dass** man verwendet als das nichtpolare Lösungsmittel einen aliphatischen oder alizyklischen C5 - C8 Kohlenwasserstoff, bzw. einen aromatischen C6 - C9 Kohlenwasserstoff, bzw. einen C4 - C8 Ether.

7. Verfahren nach Anspruch 6, **dadurch gekennzeichnet, dass** man verwendet als das nichtpolare Lösungsmittel Pentan, Hexan, Zyklohexan, Toluol oder Ethylether.

8. Verbindung *N-*[(2'-(1-Triphenylmethyl-tetrazol-5-yl)biphenyl-4-yl)methyl]-(L)-valinbenzylesterhydrochlorid der Formel III

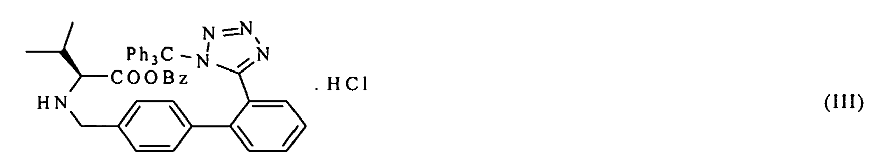

9. Verbindung nach Anspruch 8, worin die Verbindung ist im kristallinen Zustand.

## Revendications

1. Procédé de synthèse de la *N*-(1-oxopentyl)-*N*-[[2'-(1*H*-tétrazol-5-yl)[1,1'-biphényl]-4-yl]méthyl]-L-valine, connu sous la dénomination DCI valsartan, de formule I 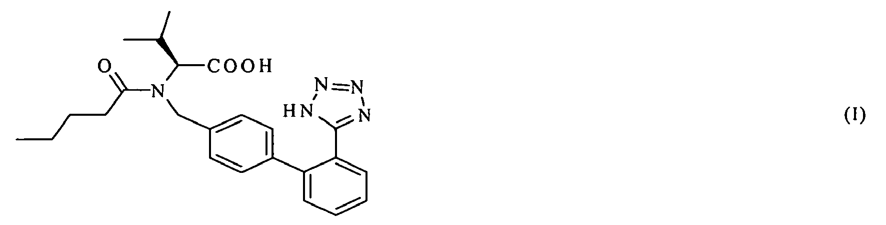 dans lequel on obtient l'ester benzylique de la *N*-[(2'-(1-triphénylméthyl-tétrazol-5-yl)biphényl-4-yl)méthyl]-(L)-valine à partir du 4-bromméthyl-2'-(1-triphénylméthyltétrazol-5-yl)biphényl par réaction avec l'ester benzylique de la L-valine, et on le transforme en valsartan par réaction avec le chlorure de valéryle et déprotection,
**caractérisé en ce que** l'on transforme l'ester benzylique de la *N*-[(2'-(1-triphénylméthyl-tétrazol-5-yl)biphényl-4-yl)méthyl]-(L)-valine de formule 7 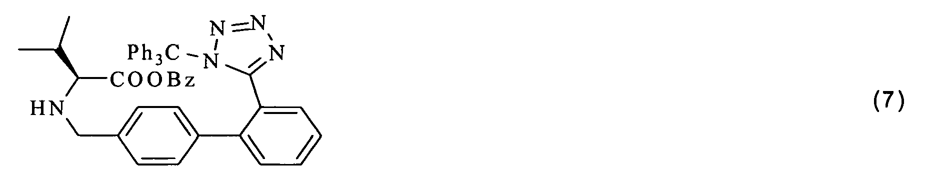 obtenu en première étape, par l'acide hydrochlorique en chlorhydrate de l'ester benzylique de la *N*-[(2'-(1-triphénylméthyl-tétrazol-5-yl)biphényl-4-yl)méthyl]-(L)-valine de formule III 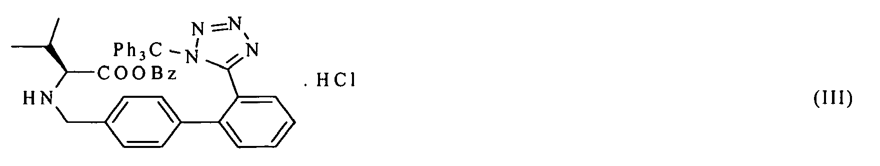 que l'on éventuellement recristallise.

2. Procédé selon la revendication 1, **caractérisé en ce que** l'on prépare le composé de formule III par acidification d'une solution de la base de l'ester benzylique de la *N*-[(2'-(1-triphénylméthyl-tétrazol-5-yl)biphényl-4-yl)méthyl]-(L)-valine en un solvant aprotique avec l'acide hydrochlorique ou le gaz chlorhydrique en simultanément extrayant le chlorhydrate résultant de formule III.

3. Procédé selon la revendication 2, **caractérisé en ce que** l'on extrait le composé de formule III à l'acétate d'éthyle et puis on le cristallise de ce solvant.

4. Procédé selon la revendication 1, **caractérisé en ce que** l'on transfert la base libre de l'ester benzylique de la *N*-[(2'-(1-triphénylmethyl-tétrazol-5-yl)biphényl-4-yl)méthyl]-(L)-valine de formule 7 dans l'acétate d'éthyle ou dans le toluène et puis on additionne un équivalent de l'acide hydrochlorique, du gaz chlorhydrique ou du gaz chlorhydrique dissout en un solvant organique, de préférence méthanol, et on cristallise le composé de formule III de ce solvant.

5. Procédé selon la revendication 3 ou 4, **caractérisé en ce que** l'on effectue la cristallisation du composé III en éliminant l'eau de la solution ou en additionnant un solvant non polaire ou en refroidissant la solution, éventuellement en combinant lesdites méthodes.

6. Procédé selon la revendication 5, **caractérisé en ce que** l'on utilise comme le solvant non polaire un hydrocarbure aliphatique ou alicyclique de C5 à C8, ou éventuellement un hydrocarbure aromatique de C6 à C9, ou éventuellement un éther de C4 à C8.

7. Procédé selon la revendication 6, **caractérisé en ce que** l'on utilise comme le solvant non polaire le pentane, l'hexane, le cyclohexane, le toluène ou l'éther éthylique.

8. Le composé, chlorhydrate de l'ester benzylique de *N*-[(2'-(1-triphénylméthyl-tétrazol-5-yl)biphényl-4-yl)méthyl]-(L)-valine, de formule III

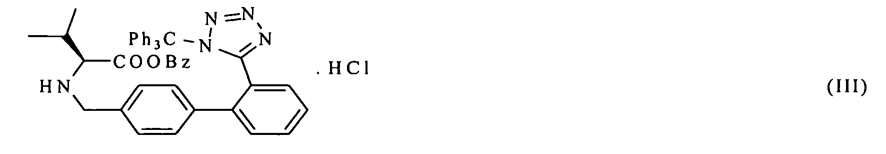

9. Le composé selon la revendication 8, où le composé est en état cristallin.
